Europäisches Patentamt

European Patent Office

Office européen des brevets

(11) Publication number: **0 107 751**
**B1**

# EUROPEAN PATENT SPECIFICATION

(45) Date of publication of patent specification: **13.05.87**

(21) Application number: **83105824.3**

(22) Date of filing: **14.06.83**

(51) Int. Cl.⁴: **A 61 K 35/02, A 61 K 33/26**

(54) Inorganic health preservative for humans.

(30) Priority: **01.10.82 JP 170923/82**

(43) Date of publication of application:
**09.05.84 Bulletin 84/19**

(45) Publication of the grant of the patent:
**13.05.87 Bulletin 87/20**

(84) Designated Contracting States:
**BE DE FR GB IT NL SE**

(56) References cited:
**GB-A-1 381 238**

**PATENTS ABSTRACTS OF JAPAN, vol. 5, no. 24(C-43)(696)**

(73) Proprietor: **Komakine, Chukei**
**13-10, Azasugidaira, Taira**
**Iwaki-shi Fukushima-ken (JP)**

(72) Inventor: **Komakine, Chukei**
**13-10, Azasugidaira, Taira**
**Iwaki-shi Fukushima-ken (JP)**

(74) Representative: **Henkel, Feiler, Hänzel & Partner**
**Möhlstrasse 37**
**D-8000 München 80 (DE)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

## Description

This invention relates to a pharmaceutical composition for preserving health whose regular daily use in small amounts improves the physical constitution of humans, helping recovery of their original health and curing diseases which have hitherto been regarded as incurable.

As set forth in the Japanese published unexamined patent application No. 150858/1980, the present inventor discovered that a material manufactured by a method similar to but not the same as that of the present invention was very effective as an antiseptic additive for general feedstuffs for domestic animals, poultry and fish. Namely, it was found that general feedstuffs mixed with 0.5 to 1.0% by weight of the above-mentioned material had the advantages that the rotting and fermentation of the aforementioned feedstuffs during storage was eliminated; the animals which took a feedstuff mixed with said material were completely free from various diseases; excrement from these animals was rendered substantially odorless; the farms could maintain a clean environment, suppressing the offensive odors which hitherto unavoidably permeated the surroundings of the farms; and the meat of the animals which took the aforesaid feedstuffs mixed with said material improved in taste. Furthermore, the amount of fat in the meat was reduced; and the death rate of the animals and their offspring were noticeably lower. Thus, livestock and poultry breeders and fishermen enjoyed great success with the above-mentioned material.

Hitherto, expensive organic synthetic sterilizers or antibiotics were applied as antiseptic additives to general feedstuffs and also as medical materials for sick animals. In recent years, however, it has been noted that such synthetic sterilizers or antibiotics lead to the growth of cancer in humans who eat the meat or eggs of animals raised on feedstuffs mixed with said antiseptic or antibiotic additives. Accordingly, the Ministry of Health and Welfare of Japan finally prohibited the addition of any of the above-mentioned organic medical materials to animal feedstuffs. At present, however, no effective substitute has been found for these organic medical materials. Consequently, feedstuff dealers, fish farmers, and animal raisers have been quite at a loss what to do. Moreover, the supervising government administration has failed to provide a proper guidance to the dealers of the aforesaid antiseptic or antibiotic materials.

In such circumstances as mentioned above, the present inventor disclosed in the aforesaid Japanese Patent Application the result of studies and experiments conducted over ten years as a feedstuff additive. This feedstuff additive has been officially tested by the Japanese government and it has been shown that it is able to eliminate the aforesaid difficulties and to realize noticeable effects. In 1982, the director of the Science and Technology Administration of the Japanese government granted a letter of commendation of the present inventor.

A product embodying the above-mentioned invention is a greyish white powdery antiseptic which is to be added to general animal feedstuffs. This product is manufactured by mixing 1 to 2 parts by weight of flyash with 8 parts by weight of crystalline ferrous sulfate heptahydrate ($FeSO_4 \cdot 7H_2O$), and drying the mixture at a temperature of 65° to 85°C for about 30 minutes while stirring to obtain shattered granules as the product.

Crystalline ferrous sulfate heptahydrate is a substantially useless, strongly acidic and deliquescent coarse granular substance having a pH value of about 3, which can be obtained as a by-product in the manufacture of titanium-white using sulfuric acid. To date, manufacturers of titanium-white have been extremely troubled by the disposal of this by-product. Hitherto, the by-product has been transported and dumped in the open sea.

When thermally dried for about 30 minutes at a temperature of 65° to 85°C, the coarse granular crystals of ferrous sulfate heptahydrate shatter. At this time, part of the water of crystallization evaporates, causing the heptahydrous crystals to turn into a stable white or faintly green shattered substance mainly consisting of ferrous sulfate monohydrate. Ferrous sulfate has long been utilized as a hematinic.

Flyash is formed of very fine globular particles obtained when the vapor from burning coal at a temperature of about 1,200°C is cooled in a flue in a coal-burning power station. The flyash is generally recovered by dust collectors. The flyash is a grayish white powdery by-product which consists of inorganic oxides activated by high temperature and is highly alkaline with a pH value estimated to be about 12. The flyash contains extremely small amounts of the oxides of P, Ca, Mg, K, Na, B, Mo, Mn, Se, Pb and Cu as the so-called minerals. Although part of the by-product flyash is sometimes used as a lubricant to be mixed with cement mortar, the greater part of it is left unused.

When the coarse crystals of ferrous sulfate heptahydrate mixed with the aforementioned percentage of flyash are thermally dried at a temperature of 65° to 85°C for about 30 minutes, the crystals are naturally shattered, causing part of the water of crystallization to be removed. Further, the surfaces of the shattered granules are covered with the fine powder of flyash, adhering to the granules. As a result, the hygroscopicity of the ferrous sulfate is prevented, thereby improving the slipperiness of the shattered granules. The particles of ferrous sulfate and those of flyash are mutually neutralized, causing the pH value of the mixed mass to be reduced to about 9 (slightly alkaline). When this slightly alkaline substance is added to animal feedstuffs, the feedstuff as a whole becomes slightly alkaline, and is rendered suitable for the preservation of the health of animals together with the effect of minerals contained in the feed.

Addition of 0.5 to 1.0% by weight of this additive to a general feedstuff has the advantage that the mixed feedstuff can be safely stored for five

2

months during the summer season, whereas general feedstuffs without said additive rot within a month's storage in the summer time. The further merits of said additive are that animals which have eaten the feedstuffs mixed with this additive have improved blood circulation, and are free from various diseases; almost all domestic animals, which have hitherto tended to become sick, can dispense with medicine; the death rate of infant pigs becomes almost zero percent; their extrement does not decompose but has a substantially odorless semisolid form, whereby the environment in which the animals are raised is greatly improved; the minerals contained in the additive enable harmful substances which develop in the animal body to be excreted therefrom; and the synergistic effects of ferrous sulfate and flyash enable the animals to have good health free from almost all diseases.

The present inventor has conceived that, if a human were to take the feed additive set forth in the Japanese published unexamined Patent Application No. 150858/1980 as a pharmaceutical human health preservative, then the same merit would be obtained as in the case of animals.

The present inventor asked many weak people to take said feed additive on a trial basis. For many years a large number of poultry and domestic animals were given the inorganic additive with their feed to ascertain the practical effect. The public agricultural experimental stations in the various districts proved the advantageous effects of the inorganic feed additive. Further, the director of the Science and Technology Agency of the Japanese Government has awarded the present inventor a prize as mentioned before in recognition of the great merit of this inorganic additive. Therefore, the present inventor entertains no anxiety about the direct application of this inorganic additive to humans.

Those who took the additive as a pharmaceutical health preservative for a few or several months praised unanimously its good effects. Moreover, no case has hitherto been cited where this health preservative had a harmful side effect during long habitual use over a period of time.

When using the additive set forth in the aforesaid Japanese Patent Application as a pharmaceutical composition for preserving the health of humans, it had some shortcomings that said agent was felt to be unpleasant at the time it was taken and is somewhat unstable because of its coarse particle formation. Therefore, the method of manufacturing said additive has been slightly modified in the present invention.

Namely, the crystals of by-product ferrous sulfate heptahydrate mixed with the prescribed percentage of flyash are thermally dried at a temperature of 65° to 85°C for about one hour. Thus, the water of crystallization contained in the ferrous sulfate heptahydrate crystals is removed to a slightly larger extent than applied before in order to increase the stability of ferrous sulfate crystals. Further, the dried mixture is finally pulverized mechanically to an extent of 100 Tyler mesh 0.149 mm or less to ensure the homogenization and stabilization of the product.

When an adult takes 0.5 to 1.0 gram of the above-mentioned slightly alkaline composition once immediately after breakfast or supper, he will be in a cheerful mood after several days and will notice that his physical strength is increased.

When the subject composition is taken orally as it is, the tongue and teeth are blackened by the effect of the ferrous component thereof, yet with no harmful results. Therefore, it is necessary to take the composition in the form of a medical wafer or enclosed in a capsule. Further, the excrement of a person who has taken this composition turns jet-blacks with no harmful effect. Since, however, the appearance of this black color results from the complete excretion of injurious substances retained in the human body, it is unnecessary to feel any anxiety about this event. The cessation of application of the preservative eliminates immediately the black color from the excrement.

If a person takes this pharmaceutical composition continuously for at least two or three months, his constitution, even if abnormal, can be restored to its original healthy state. Moreover, the injurious substances remaining in his body are all excreted as mentioned afore, resulting from the effects of minerals and ferrous sulfate contained in this health preservative.

Consequently, anyone who has taken this pharmaceutical composition will be free from various diseases. As can be seen from the following examples, miraculous phenomena have taken place such as the composition of this application completely curing various diseases which have been regarded as incurable by present day medical science, as, for example, chronic hepatitis, allergic diseases, insomnia, polyuria, auricular fibrillation, empyema, hypertension and hyperglycemia.

One instance was reported where even a cancer was alleviated. Though it may be hasty to conclude from this single piece of evidence that the present pharmaceutical composition is effective as an anti-cancer substance, yet it is not necessarily impossible that said agent will be sufficiently improved in the future to be applied as an anti-cancer agent. The present inventor believes that, at least, the present pharmaceutical composition can be used as a cancer-preventing agent. At present, a certain medical college is entrusted with research work in this particular field.

When anyone who feels that his constitution is abnormal, takes the present pharmaceutical composition, his unusual constitution will be restored to its original healthy state. Ailments other than virus diseases results from the unusual condition of the constitution of human body. For instance, when anyone feels fatigue, shoulder stiffness or lumbago, the intake of the present pharmaceutical composition for several days effectively relieves him from such ailments, completely eliminating the necessity of resorting to acupuncture. Further, the continued intake of this pharmaceutical composition prevents a healthy man from contracting malignant diseases. Since the present pharmaceutical composition does not contain any harmful organic chemical compounds, the long continuous application of this composition does not cause any undesirable side effect at all.

Furthermore, the pharmaceutical composition of this invention is characterized in that it can be commercially manufactured by a simple process at a very low cost from useless industrial byproducts which have been proved to be hygienically safe.

Sometimes the application of the present pharmaceutical composition for about two weeks temporarily causes the user to suffer from a slightly abnormal constitution, for instance, diarrhoea, as in the case with common Chinese medicines or with known health preservative prepared from chlorophyta, for example, chlorella. However, this phenomena is a positive proof that the present composition is effective. Therefore, the user should take the composition continuously without any fear.

The effects of the pharmaceutical composition of this invention will become more apparent with reference to the examples which follow.

### Example 1

A 74-year-old man had been troubled by insomnia for about 20 years. His physician administered continuously a psychosomatic stabilizer to the patient. Three years ago, he began to feel to pain in the articulations of knees and toes, and finally could not walk without a walking stick.

In the meanwhile, he learned by chance of the present composition, and he took one gram of the composition after breakfast every day. In two months, he slept well and found it unnecessary to take any psychosomatic stabilizers, and moreover he was relieved from pain in the joints and could walk without a walking stick.

### Example 2

A 65-year-old man, suffering from pollakisuria for several years, was finally forced to wake up every hour during the night to urinate. Three months after the intake of the present composition, he only had to urinate every four or five hours.

### Example 3

A 35-year-old woman, who suffered from water eczema appearing on the soles of her feet every summer, applied a drug to the defective spots, but without success. However, the intake of the present pharmaceutical composition for several days cured substantially the water eczema. The subsequent continuous application of said composition for two months eliminated completely the water eczema from the soles of her feet.

### Example 4

The room of a bed-ridden woman 85 years old suffering from senility was permeated with her obnoxious body and excrement odors which bothered the nurse. When, however, the present composition was administered to her, the medical care room was substantially freed from stinking odors, facilitating her nursing. Before the intake of said composition, she often suffered from bedsores on her back. Two months after the intake of said composition, she recovered her physical strength and could move her limbs by herself little by little. As a result, she was freed from bedsores.

### Example 5

A 70-year-old man, who had suffered from auricular fibrillation for 15 years, always had a pulse of over 85 beats per minute, and was hospitalized for 50 days, but without success. After leaving the hospital, he began to take the present composition. In two months, his pulse rate was reduced to about 70 beats per minute, and he was substantially relieved from the auricular fibrillation.

Moreover, the old man sometimes had a fit of cardiac asthma and was medically examined and received medication twice a month from a physician. Four months after the intake of the present pharmaceutical composition, no cardiac asthma took place. Before the intake of the composition, his blood pressure was 170 to 180 systolic and 100 to 110 dystolic. However, three months after the intake of the pharmaceutical composition, his blood pressure was restored to a normal level, i.e. 140 to 150 over 80 to 90.

### Example 6

A 49-year-old manager of a bank branch office suffered greatly from physical stress due to his extremely high work load. Three weeks after the intake of the present pharmaceutical composition, however, he felt no fatigue and recovered his sexual appetite which has previously been at an ebb.

### Example 7

A 25-year-old man underwent two surgical operations for empyema, but without success. He continued to be troubled as much as before by the constant ejection of green stinking nasal mucus. He suffered from a stuffy nose and headache and felt a decline in his vitality. Three months after the intake of the present pharmaceutical composition, however, he recovered completely from the above-mentioned ailments and enjoyed a pleasant feeling.

### Example 8

The present pharmaceutical composition was administered to six languid hospitalized pregnant women. About two weeks after administration, they recovered their vigor and were successfully delivered at full term. Three boys and three girls thus born grew into such robust babies as could hardly be expected from the previously hospitalized sick women. The babies had better pulses and greater body-weight than normal.

### Example 9

A 51-year-old woman, who suffered from neuralgia after leaving the hospital where she was operated on for cancer of the breast, underwent acupuncture, but without success. Three months after the intake of the present pharmaceutical composition, she was little bothered by neuralgia, and she recovered her physical strength, becoming more portly than before.

### Example 10

A 75-year-old man, who had continuously all day long read small letter literatures such as patent publications and prepared manuscripts for more than 20 years, grew noticeably senile about two years ago and had weakened eyesight. In the evening he became nearly blind, failing to clearly decipher small letters.

Two months after the intake of the present pharmaceutical composition, he recovered his eyesight and felt no fatigue even when reading or writing all day long. He could clearly distinguish outdoor scenery. His hair which had turned gray two years ago became almost black three months after the intake of said health composition. Miraculously, white hair substantially disappeared from his head.

### Example 11

A 62-year-old man, who had been afflicted since birth with perspiring palms which remained wet even during the winter time, always suffered from a complex that his defective palms might cause a person shaking hands with him to feel unpleasant. Two months after the intake of the present pharmaceutical composition, the perspiration of his palms was completely eliminated, enabling him to calmly shake hands with other people.

### Example 12

A 64-year-old man, who had been afflicted with indisposition due to the occurrence of nauseous, languidness and occasional fever, was examined by a physician. As a result, it was found that he was suffering from chronic hepatitis, with the GOT and GPT values respectively determined to be 150 and the overall cholesterol value to be 360. The physician told him that no suitable medical treatment was currently available for chronic hepatitis, and he should advisably refrain from overwork in order to suppress the further development of said chronic hepatitis. The patient began to take in the present pharmaceutical composition upon recommendation of an acquaintance. The physician's examination four months after the intake of said composition indicated that the GOT value fell to 35, the GPT value to 40 and the overall cholesterol value to 185, proving that the patient had recovered from chronic hepatitis. Surprised at this event, the physician said that the cure was really miraculous. For reference, healthy persons have generally GOT value of 80 to 40, GPT value of 5 to 35 and overall cholesterol value of 130 to 250.

### Example 13

A 39-year-old man, who had been seriously injured in a motor accident, underwent blood transfusion during the surgical operation. Unfortunately, the blood transfusion led to the occurrence of acute serum hepatitis after three years. The serum hepatitis, which appeared to be cured at one time, lapsed into chronic serum hepatitis two years later, with the ZTT value determined to be 18. Then, months after the intake of the present pharmaceutical composition, however, the GOP value fell to 22, the GPT value to 36 and the ZTT value to 7. It was ascertained that he had recovered from the chronic serum hepatitis. For reference, a healthy man has usually a ZTT value lower than 12.

### Example 14

A 48-year-old man who felt abnormalities in the mouth was examined by a physician. As a result, it was found that a cancerous black larynx tumor and tongue cancer were growing concurrently. The man, whose internal organs indicated no abnormality, underwent medical treatment as a day patient. During said medical treatment, the patient took secretly the present pharmaceutical composition. Three months later, little pain was felt in the tongue and larynx while eating, and the color of larynx tumor faded. Four months after the intake of the health preservative, no abnormalities were felt in the mouth. The patient increased in the physical strength and improved complexion. The physician assured him that recovery to such an extent made it unecessary to fear the return of cancer.

### Example 15

A 59-year-old man who had been afflicted with allergic nettle rash for about 20 years never failed to suffer from nettle rash when he ate shrimp, crab, egg, milk, sardine, mackeral, octopus or mackeral-pike. Therefore, he ceased to eat any of these foods. Later, he happened to be informed of the meritorious effect of the present pharmaceutical composition. He took it for trial for some time. About 40 days later, he was forced to eat chicken and egg with rice. The next morning no nettle rash appeared. Encouraged by this event, he continued to take the present composition. Two months after the initial intake, he was completely free from nettle rash even when he ate the above listed allergenic foods.

### Example 16

A 36-year-old woman, who had suffered from anemia for a long time and who had a systolic blood pressure of 70 to 80 and dystolic blood pressure of 50 to 60, usually felt dizzy and unstable when walking. The intake of the present pharmaceutical composition for more than three months improved her blood pressure to the normal level, that is, about 120 over 80. She could sleep soundly at night, was cured of constipation, and had restored vigor.

Example 17

A 56-year-old man, who had been afflicted with an unhealthy condition for about 15 years, was examined by a physician. As a result, it was found that he suffered from diabetes with the blood sugar concentration determined to be about 300. (A blood sugar concentration of 350 endangers human life.) He was temporarily hospitalized to undergo insulin treatment and diet treatment, but without success. Later, he relied on natural recuperation at home. Then, he chanced to take the present pharamceutical composition. In about two months, the blood sugar concentration fell to below 150. (The standard blood sugar concentration ranges between 100 and 150.) His diet was restored to ordinary foods, making it unnecessary to inject insulin. His blood pressure which previously ranged from 180 to 190 fell to 130 to 120. He was free from pain in the glans of the penis peculiar to sufferers of diabetes, and could enjoy sexual intercourse.

**Claims**

1. A pharmaceutical composition for preserving the health of humans which is prepared by mixing 8 parts by weight of crystalline ferrous sulfate heptahydrate with 1 to 2 parts by weight of flyash; drying the mixture for about one hour at a temperature of 65° to 85°C to obtain shattered granules; and pulverizing mechanically said shattered granules into a finely powdered substance.

2. The pharmaceutical composition according to claim 1, characterized in that the crystalline ferrous sulfate heptahydrate is a by-product obtained from a titanium-white manufacturing plant using sulfuric acid.

3. The pharmaceutical composition according to claim 1, characterized in that the flyash is a by-product fine powder obtained from a steam power station.

4. The pharmaceutical composition according to claim 1, characterized in that the degree of pulverization of the final product is about 100 Tyler mesh (0.149 mm).

**Patentansprüche**

1. Arzneimittel zur Bewahrung der menschlichen Gesundheit, hergestellt durch Vermischen von 8 Gew.-Teilen kristallinen Eisen(II)-sulfat-heptahydrats mit 1 bis 2 Teil(en) Flugasche, etwa 1-stündiges Trocknen des Gemischs bei einer Temperatur von 65—85°C zur Bildung .von Granulattrümmern und mechanisches Pulverisieren der Granulattrümmer zu einer feinpulverigen Substanz.

2. Arzneimittel nach Anspruch 1, dadurch gekennzeichnet, daß das kristalline Eisen(II)-sulfat-heptahydrat aus einem in einer mit Schwefelsäure arbeitenden Titanweiß-Herstellungsanlage angefallenen Nebenprodukt besteht.

3. Arzneimittel nach Anspruch 1, dadurch gekennzeichnet, daß die Flugasche aus einem in einem Dampfkraftwerk angefallenen feinpulverigen Nebenprodukt besteht.

4. Arzneimittel nach Anspruch 1, dadurch gekennzeichnet, daß der Pulverisierungsgrad des Endprodukts etwa 100 Tyler mesh (0,149 mm) beträgt.

**Revendications**

1. Une composition pharmaceutique pour conserver la santé des humains qui est préparée en mélangeant 8 parties en poids de sulfate ferreux heptahydraté avec 1 à 2 parties en poids de cendre volante; à sécher le mélange pendant environ 1 h pendant une température de 65 à 85°C pour obtenir des granules désagrégés; et à pulvériser mécaniquement ces granules désagrégés en une substance finement pulvérisée.

2. La composition pharmaceutique selon la revendication 1, caractérisée en ce que le sulfate ferreux heptahydraté cristallin est un sous-produit obtenu dans une usine de fabrication de blanc de titane utilisant l'acide sulfurique.

3. La composition pharmaceutique selon la revendication 1, caractérisée en ce que la cendre volante est un sous-produit en poudre fine obtenu dans une centrale thermique à vapeur.

4. La composition pharmaceutique selon la revendication 1, caractérisée en ce que le degré de pulvérisation du produit final correspond environ à un tamis de maille 100 Tyler (0,149 mm).